(19) Europäisches Patentamt
European Patent Office
Office européen des brevets

(11) Veröffentlichungsnummer: **0 023 326**
**B1**

# (12) EUROPÄISCHE PATENTSCHRIFT

(45) Veröffentlichungstag der Patentschrift: **26.05.82**

(51) Int. Cl.³: **C 07 D 231/18,** A 01 N 47/18

(21) Anmeldenummer: **80104235.9**

(22) Anmeldetag: **18.07.80**

(54) **N-Methyl-0-pyrazol(4)yl-carbaminsäureester, Verfahren zu ihrer Herstellung und ihre Verwendung als Schädlingsbekämpfungsmittel.**

(30) Priorität: **31.07.79 DE 2931033**

(43) Veröffentlichungstag der Anmeldung: **04.02.81 Patentblatt 81/5**

(45) Bekanntmachung des Hinweises auf die Patenterteilung: **26.05.82 Patentblatt 82/21**

(84) Benannte Vertragsstaaten: **AT BE CH DE FR GB IT LI NL SE**

(56) Entgegenhaltungen:
CH-A-414244
DE-A-2144124
DE-A-2420360

(73) Patentinhaber: **BAYER AG, Zentralbereich Patente, Marken und Lizenzen, D-5090 Leverkusen 1, Bayerwerk (DE)**

(72) Erfinder: **Maurer, Fritz, Dr., Roeberstrasse 8, D-5600 Wuppertal 1 (DE)**
Erfinder: **Hammann, Ingeborg, Dr., Belfortstrasse 9, D-5000 Koeln 1 (DE)**
Erfinder: **Homeyer, Bernhard, Dr., Obere Strasse 28, D-5090 Leverkusen 3 (DE)**
Erfinder: **Behrenz, Wolfgang, Dr., Untergründemich 14, D-5063 Overath (DE)**

**EP 0 023 326 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# N-Methyl-O-pyrazol(4)yl-carbaminsäureester, Verfahren zu ihrer Herstellung und ihre Verwendung als Schädlingsbekämpfungsmittel

Die Erfindung betrifft neue N-Methyl-O-pyrazol(4)yl-carbaminsäureester, ein Verfahren zu ihrer Herstellung und ihre Verwendung als Schädlingsbekämpfungsmittel, insbesondere als Insektizide, Akarizide und Nematizide.

Es ist bekannt, dass bestimmte Carbaminsäureester, wie z.B. N,N-Dimethyl-O-(1-iso-propyl-3-methyl-pyrazol(5)yl)-carbaminsäureester, N,-Methyl-O-(2-iso-propoxy-phenyl)-carbaminsäureester und N-Methyl-O-(2,3-dihydro-2,2-dimethyl-7-benzofuranyl)-carbaminsäureester, als Pestizide wirksam sind (vgl. CH-PS 282655, DE-AS 1108202, US-PS 3474171). Die Wirkung dieser Verbindungen ist jedoch, insbesondere bei niedrigen Wirkstoffkonzentrationen und Aufwandmengen, nicht immer ganz zufriedenstellend.

Aus der CH-PS 414249 ist 1-Phenyl-4-hydroxy-pyrazol sowie seine Verwendung zur Herstellung insektizider Carbamate bekannt. Die daraus hergstellten Carbamate befriedigen jedoch hinsichtlich ihrer Wirkung nicht immer.

Es wurden nun neue N-Methyl-O-pyrazol(4)yl-carbaminsäureester der Formel I

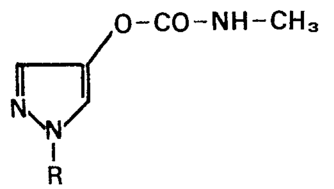

(I)

gefunden, in welcher

R für geradkettiges oder verzweigtes Alkyl, Alkenyl, Alkinyl oder Alkoxyalkyl mit jeweils bis zu 4 C-Atomen im Alkylteil oder für Cycloalkyl mit 3-6 C-Atomen steht.

Man erhält die neuen Carbaminsäureester der Formel (I), wenn man in 4-Hydroxypyrazole der Formel II

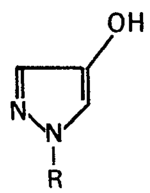

(II)

in welcher

R die oben angegebene Bedeutung hat, in üblicher Weise die N-Methylcarbamoylgruppe einführt.

Die neuen N-Methyl-O-pyrazol(4)yl-carbaminsäureester der Formel (I) zeichnen sich durch hohe Wirksamkeit als Schädlingsbekämpfungsmittel, insbesondere durch hohe insektizide, akarizide und nematizide Wirksamkeit aus.

Überraschenderweise zeigen die erfindungsgemässen Verbindungen der Formel (I) erheblich höhere insektizide, akarizide und nematizide Wirkung als bekannte Verbindungen ähnlicher Konstitution und gleicher Wirkungsrichtung.

Verwendet man als Ausgangsstoffe beispielsweise 1-Cyclobutyl-4-hydroxypyrazol und Methylisocyanat, so kann die Reaktion dieser Verbindungen durch folgendes Formelschema skizziert werden:

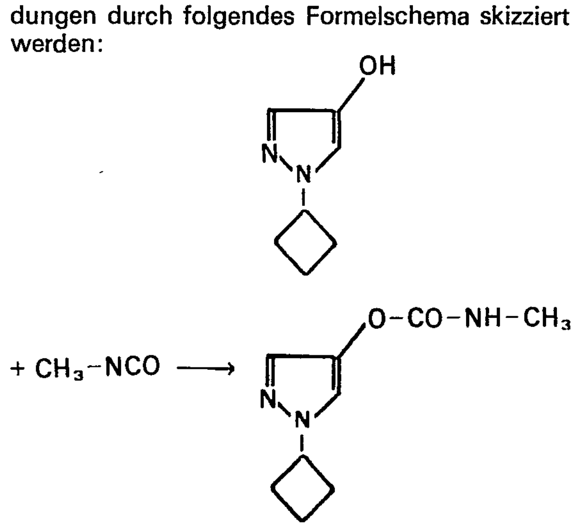


Die als Ausgangsstoffe zu verwendenden 4-Hydroxypyrazole sind durch Formel (II) definiert. Vorzugsweise steht darin

R für geradkettiges oder verzweigtes Alkyl mit 1 bis 4 Kohlenstoffatomen oder für Cycloalkyl mit 3 bis 6 Kohlenstoffatomen.

Als Beispiele seien genannt:

1-Methyl-, 1-Ethyl-, 1-n-Propyl-, 1-iso-Propyl-, 1-n-Butyl-, 1-iso-Butyl-, 1-sek.-Butyl-, 1-tert.-Butyl-, 1-Cyclopropyl-, 1-Cyclobutyl-, 1-Cyclopentyl- und 1-Cyclohexyl-4-hydroxy-pyrazol.

Die 4-Hydroxypyrazole der Formel (II) sind teilweise bekannt (vergleiche „Liebigs Ann. Chem." *313* (1900), S. 17). Man erhält sie beispielsweise durch Umsetzung von bekannten 4-Methoxypyrazolen mit Bromwasserstoffsäure. Die Herstellung der 4-Methoxypyrazole erfolgt auf bekannte Weise aus Hydrazinen und 2-Methoxy-4-dimethylamino-acrolein (vergleiche „Archiv der Pharmazie" *300* (1967), S. 704-708).

Das als weitere Ausgangsverbindung zu verwendende Methylisocyanat ist bekannt.

Das Verfahren zur Herstellung der erfindungsgemässen N-Methyl-O-pyrazol(4)yl-carbaminsäureester wird bevorzugt unter Verwendung geeigneter Lösungs- oder Verdünnungsmittel durchgeführt. Als solche kommen praktisch alle inerten organischen Solventien in frage. Hierzu gehören insbesondere aliphatische und aromatische, gegebenenfalls chlorierte Kohlenwasserstoffe, wie Benzin, Benzol, Toluol, Xylol, Methylenchlorid, Chloroform, Tetrachlorkohlenstoff, Chlorbenzol und o-Dichlorbenzol, Ether wie Diethyl- und Dibutylether, Tetrahydrofuran und Dioxan, Ketone wie Aceton, Methylethyl-, Methylisopropyl- und Methylisobutylketon sowie Nitrile wie Acetonitrile und Propionitril.

Das Herstellungsverfahren wird gegebenfalls unter Verwendung eines Katalysators durchgeführt. Als Katalysatoren können hierbei insbesondere aliphatische, aromatische oder heterocyclische Amine, wie z.B. Triethylamin, Trimethylamin,

Dimethylanilin, Dimethylbenzylamin, Pyridin, Diazabicyclooctan, Diazabicyclononan und Diazabicycloundecen verwendet werden.

Die Reaktionstemperatur kann innerhalb eines grösseren Bereichs variiert werden. Im allgemeinen arbeitet man zwischen 0 und 100°C, vorzugsweise zwischen 10 und 80°C.

Das Verfahren wird im allgemeinen bei Normaldruck durchgeführt. Zur Durchführung des erfindungsgemässen Verfahrens setzt man auf 1 Mol 4-Hydroxy-pyrazol der Formel (II) gewöhnlich zwischen 1 und 1,5 Mol, vorzugsweise zwischen 1 und 1,2 Mol Methylisocyanat ein. Die Umsetzung wird vorzugsweise unter Verwendung eines der oben genannten Katalysatoren in einem der oben angegebenen Verdünnungsmittel durchgeführt. Das Reaktionsgemisch wird mehrere Stunden bei der erforderlichen Temperatur gerührt. Dann wird das Lösungsmittel im Vakuum abdestilliert. Man erhält so die Produkte in öliger oder kristalliner Form. Zur Charakterisierung dient der Schmelzpunkt oder der Brechungsindex.

Andere Verfahren zur Einführung der N-Methylcarbamoylgruppe wären die Umsetzung der Verbindung II mit Phosgen und anschliessende Reaktion der entstandenen Verbindungen mit Methylamin oder die Umsetzung der Verbindung II mit ω-Methylcarbamidsäurechlorid.

Die erfindungsgemässen N-Methyl-O-pyrazol (4)yl-carbaminsäureester zeichnen sich, wie bereits erwähnt, durch hervorragende insektizide, akarizide und nematizide Wirksamkeit aus. Bei geringer Phytotoxizität wirken sie gegen Pflanzenschädlinge wie auch gegen Hygiene- und Vorratsschädlinge. Daher können die erfindungsgemässen Verbindungen mit Erfolg im Pflanzenschutz und Vorratsschutz sowie im Hygienebereich als Schädlingsbekämpfungsmittel eingesetzt werden.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp.

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprio spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp.,

Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp.

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Zu den Pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp.

Die Wirkstoffe können in die üblichen Formulierungen übergeführt werden, wie Lösungen, Emulsionen, Suspensionen, Pulver, Schäume, Pasten, Granulate, Aerosole, Wirkstoffimprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebelformulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphtaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosoltreibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z.B. nichtionogene und anionische Emulgatoren, wie Polyoxyäthylenfettsäure-ester, Polyoxyäthylenfettalkoholäther, z.B. Alkylarylpolyglykoläther, Alkylsulfonate, Alkylsulfate, Arylsulfonate sowie Eiweisshydrolysate; als Dispergiermittel kommen in Frage: z.B. Lignin-sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azolmetallphtalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.% Wirkstoff, vorzugsweise zwischen 0,5 und 90%.

Die Anwendung der erfindungsgemässen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.% Wirkstoff, vorzugsweise zwischen 0,0001 und 1 Gew.% liegen.

Die Anwendung geschieht in einer den Anwendungsformen angepassten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemässen Wirkstoffe eignen sich auch zur Bekämpfung von Ekto- und Endoparasiten auf dem Veterinärmedizinischen Gebiet.

Die Anwendung der erfindungsgemässen Wirkstoffe geschieht im Veterinärsektor in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgiessens (pour-on and spot-on) und des Einpuderns sowie durch parenterale Anwendung in Form beispielsweise der Injektion.

*Beispiel A:*

Plutella-Test

Lösungsmittel: 3 Gewichtsteile Dimethylformamid

Emulgator: 1 Gewichtseil Alkylarylpolyglykoläther

Zur Herstellung einer zweckmässigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100%, dass alle Raupen abgetötet wurden; 0% bedeutet, dass keine Raupen abgetötet wurden.

Bei diesem Test zeigen z.B. die folgenden Verbindungen der Herstellungsbeispiele überlegene Wirksamkeit gegenüber dem Stand der Technik: 3, 2, 4, 1, 5, 6.

*Beispiel B:*

Tetranychus-Test (resistent)

Lösungsmittel: 3 Gewichtsteile Dimethylformamid

Emulgator: 1 Gewichtsteil Alkylarylpolyglykoläther

Zur Herstellung einer zweckmässigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100%, dass alle Spinnmilben abgetötet wurden; 0% bedeutet, dass keine Spinnmilben abgetötet wurden.

Bei diesem Test zeigen z.B. die folgenden Verbindungen der Herstellungsbeispiele überlegene Wirksamkeit gegenüber dem Stand der Technik: 5, 4, 3.

*Beispiel C:*

Grenzkonzentrations-Test/Wurzelsystemische Wirkung

Testinsekt: Phaedon cochleariae

Lösungsmittel: 3 Gewichtsteile Aceton

Emulgator: 1 Gewichtsteil Alkylarylpolyglykoläther

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (=mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschliesslich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100%, wenn alle Testtiere abgetötet sind und 0%, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 1.

*Beispiel D:*

Grenzkonzentrations-Test/Nematoden

Testnematode: Meloidogyne incognita

Lösungsmittel: 3 Gewichtsteile Aceton

Emulgator: 1 Gewichtsteil Alkylarylpolyglykoläther

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt, der mit den Testnematoden stark verseucht ist. Dabei spielt die Konzentration des Wirkstofs in der Zubereitung praktisch keine Rolle, entscheidend ist allein die Wirkstoffmenge pro Volumeneinheit Boden, welche in ppm angegeben wird. Man füllt den behandelten Boden in Töpfe, sät Salat ein und hält die Töpfe bei einer Gewächshaus-Temperatur von 27°C.

Nach 4 Wochen werden die Salatwurzeln auf Nematodenbefall (Wurzelgallen) untersucht und der Wirkungsgrad des Wirkstoffs in % bestimmt. Der Wirkungsgrad ist 100%, wenn der Befall vollständig vermieden wird, er ist 0%, wenn der Befall genau so hoch ist wie bei den Kontrollpflanzen in unbehandeltem, aber in gleicher Weise verseuchtem Boden.

Bei diesem Test zeigen z.B. die folgenden Verbindungen der Herstellungsbeispiele überlegene Wirksamkeit gegenüber dem Stand der Technik: 1.

*Beispiel E:*

$LT_{100}$-Test für Dipteren

Testtiere: Aedes aegypti

Zahl der Testtiere: 25

Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten geringeren Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro m² Filterpapier verschieden hoch. Anschliessend gibt man die angegebene Anzahl der Testtiere in die Petrischale und bedeckt sie mit einem Glasdeckel.

Der Zustand der Testtiere wird laufend kontrolliert. Es wird diejenige Zeit ermittelt, welche für einen 100 %igen knock down-Effekt notwendig ist.

Bei diesem Test zeigen z.B. die folgenden Verbindungen der Herstellungsbeispiele überlegene Wirkung gegenüber dem Stand der Technik: 2, 4, 1, 5, 6.

*Herstellungsbeispiele*

*Beispiel 1:*

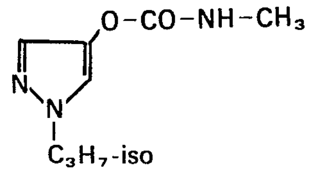

Zu einer Lösung von 12,6 g (0,1 Mol) 1-Isopropyl-4-hydroxypyrazol in 100 ml Aceton und 2 Tropfen Triethylamin gibt man 6 g (0,105 Mol) Methylisocyanat. Das Gemisch wird 12 h bei 45°C gerührt, dann destilliert man das Lösungsmittel im Vakuum ab. Man erhält so 17,4 g (95% der Theorie) N-Methyl-O-(1-isopropylpyrazol(4)yl)-carbaminsäureester in Form beiger Kristalle mit dem Schmelzpunkt 40°C.

Analog Beispiel 1.1 können die folgenden Verbindungen der Formel

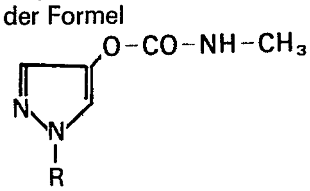

hergestellt werden:

| Beispiel Nr. | R | Ausbeute (% der Theorie) | Brechungsindex; Schmelzpunkt (°C) |
|---|---|---|---|
| 2 | $C_2H_5$ | 77 | $n_D^{22}$:1,4998 |
| 3 | $CH_3$ | 67 | 62 |
| 4 | $C_3H_7$-n | 90 | $n_D^{22}$:1,4963 |
| 5 | $C_4H_9$-sek. | 79 | $n_D^{22}$:1,4928 |
| 6 | 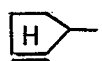 | 94 | 115 |
| 7 | 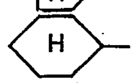 | | |
| 8 | 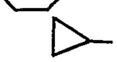 | | |
| 9 | $C_4H_9$-n | 93 | 51 |
| 10 | $C_4H_9$-iso | 95 | 80 |
| 11 | $C_4H_9$-tert. | 95 | 71 |
| 12 | $CH_2=CH-CH_2-$ | 95 | $n_D^{23}$:1,5100 |
| 13 | $CH\equiv C-CH_2-$ | | |
| 14 | $C_2H_5-O-C_2H_4-$ | 98 | $n_D^{23}$:1,4915 |

Die als Ausgangsmaterialien zu verwendenden 4-Hydroxypyrazole können z.B. wie folgt hergestellt werden:

*Beispiel 2.1:*

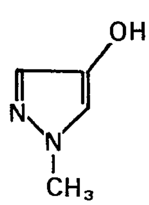

Eine Lösung von 22,4 g (0,2 Mol) 1-Methyl-4-methoxypyrazol (Darstellung s. „Archiv der Pharmazie" *300* (1967), S. 704-708) in 100 ml 48%iger Bromwasserstoffsäure wird 9 h unter Rückfluss gekocht. Dann destilliert man die überschüssige Säure im Vakuum ab, löst den Rückstand in 40 ml Wasser und neutralisiert die Lösung durch Zugabe von festem Natriumhydrogencarbonat. Anschliessend extrahiert man 6mal mit je 40 ml Chloroform, trocknet die vereinigten Auszüge über Natriumsulfat und destilliert das Lösungsmittel im Vakuum ab. Zurück bleiben 8,2 g (42% der Theorie) 1-Methyl-4-hydroxypyrazol in Form schwach gelber Kristalle mit dem Schmelzpunkt 71°C.

In analoger Weise können die folgenden Verbindungen der Formel

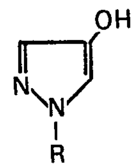

herstellt werden:

| Beispiel Nr. | R | Ausbeute (% der Theorie) | Brechungsindex; Schmelzpunkt (°C) |
|---|---|---|---|
| 2.2 | $C_2H_5$ | 65 | $n_D^{20}$:1,5078 |
| 2.3 | $C_3H_7$-n | 91 | $n_D^{21}$:1,5022 |
| 2.4 | $C_3H_7$-iso | 75 | 63 |
| 2.5 | $C_4H_9$-sek. | 91 | $n_D^{21}$:1,4994 |
| 2.6 | 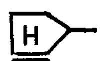 | 62 | 79 |
| 2.7 | 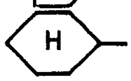 | | |
| 2.8 | 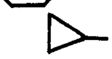 | | |
| 2.9 | $C_4H_9$-n | 88 | $n_D^{20}$:1,5014 |
| 2.10 | $C_4H_9$-iso | 78 | 32 |
| 2.11 | $C_4H_9$-tert. | 50 | 89 |
| 2.12 | $CH_2=CH-CH_2-$ | 73 | $n_D^{20}$:1,5163 |
| 2.13 | $CH\equiv C-CH_2-$ | | |
| 2.14 | $C_2H_5OC_2H_4-$ | 50 | $n_D^{20}$:1,5015 |

Die teilweise bekannten 4-Methoxypyrazole, die als Vorprodukte für die 4-Hydroxypyrazole eingegesetzt werden, können z.B. wie folgt hergestellt werden:

*Beispiel 3.1:*

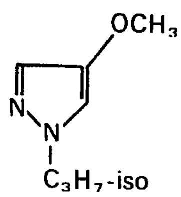

Ein Gemisch aus 129 g (1 Mol) 2-Methoxy-3-dimethylaminoacrolein (Darstellung s. „Archiv der Pharmazie" *300* (1967), S. 704-708), 123 g (1 Mol) Isopropylhydrazinhemisulfat und 1,1 Mol einer Lösung von Natriummethylat in 400 ml Methanol wird 24 h unter Rückfluss gekocht. Dann destilliert man das Lösungsmittel im Vakuum ab, versetzt den Rückstand mit 500 ml Wasser und extrahiert dann mit 1 l Chloroform. Die organische Lösung wird mit 500 ml Wasser gewaschen, über Natriumsulfat getrocknet und dann bei 30°C im Vakuum eingedampft. Den Rückstand destilliert man im Vakuum und erhält so 82,5 g (60% der Theorie) 1-Isopropyl-4-methoxypyrazol in Form eines gelben Öles mit dem Siedepunkt 94-96°C/14 Torr.

*Beispiel 3.2:*

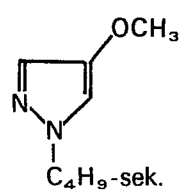

Ein Gemisch aus 23,6 g (0,21 Mol) Kalium-tert.-butylat, 19,6 g (0,2 Mol) 4-Methoxypyrazol (Darstellung s. „Archiv der Pharmazie" *300* (1967), S. 704-708), 100 ml Tetrahydrofuran und 28,7 g (0,21 Mol) sek.-Butylbromid wird 18 h bei 70°C gerührt. Dann gibt man 3000 ml Wasser zu und extrahiert 3mal mit je 100 ml Chloroform. Die vereinigten organischen Phasen werden über Natriumsulfat getrocknet und im Vakuum eingedampft. Aus dem Rückstand erhält man durch Vakuumdestillation 13,2 g (42% der Theorie) 1-sek.-Butyl-4-methoxypyrazol in Form eines farblosen Öles mit dem Siedepunkt 60°C/0,2 Torr.

Analog einem der Beispielle 3.1 bzw. 3.2 können die folgenden Verbindungen der Formel

hergestellt werden:

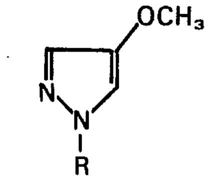

| Beispiel Nr. | R | Ausbeute (% der Theorie) | Brechungsindex; Siedepunkt (°C/Torr) |
|---|---|---|---|
| 3.3 | $CH_3$ | 80 | 72-74/7 |
| 3.4 | $C_2H_5$ | 54 | 90-93/14 |
| 3.5 | $C_3H_7$-n | 75 | 88-91/7 |
| 3.6 | 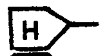 | 67 | 120/7 |
| 3.7 | 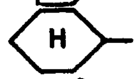 | | |
| 3.8 | 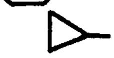 | | |
| 3.9 | $C_4H_9$-n | 87 | 54/0,1 |
| 3.10 | $C_4H_9$-iso | 65 | 53/0,3 |
| 3.11 | $C_4H_9$-tert. | 65 | 110-112/10 |
| 3.12 | $CH_2=CH-CH_2-$ | | |
| 3.13 | $CH\equiv C-CH_2-$ | | |
| 3.14 | $C_2H_5-O-C_2H_4-$ | | |

**Patentansprüche**

1. N-Methyl-O-pyrazol(4)yl-carbaminsäureester der Formel (I)

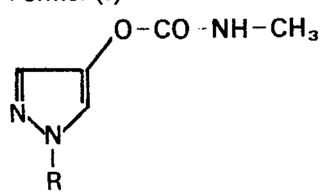

(I)

in welcher
R für geradkettiges oder verzweigtes Alkyl, Alkenyl, Alkinyl, Alkoxyalkyl mit jeweils bis zu 4 C-Atomen im Alkylteil oder für Cycloalkyl mit 3-6 C-Atomen steht.

2. Verbindung gemäss Anspruch 1 der Formel

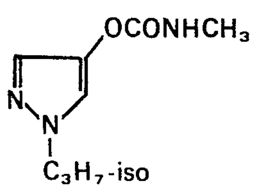

3. Verbindung gemäss Anspruch 1 der Formel

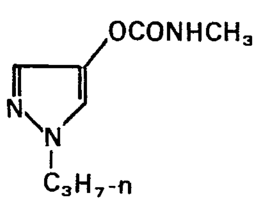

4. Verfahren zur Herstellung der Carbaminsäureester der Formel (I)

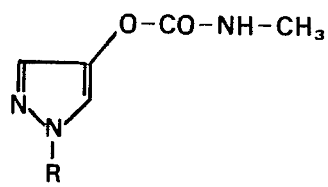

(I)

in welcher
R für geradkettiges oder verzweigtes Alkyl, Alkenyl, Alkinyl, Alkoxyalkyl mit jeweils bis zu 4 C-Atomen im Alkylteil oder für Cycloalkyl mit 3-6 C-Atomen steht,
dadurch gekennzeichnet, dass man in 4-Hydroxypyrazole der Formel (II)

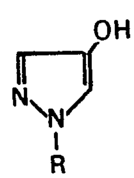

(II)

in welcher
R die oben angegebene Bedeutung hat, in üblicher Weise die N-Methylcarbamoylgruppe einführt.

5. Schädlingsbekämpfungsmittel gekennzeichnet durch einen Gehalt an mindestens einem N-Methyl-O-pyrazol(4)yl-carbamidsäureester der Formel (I).

6. Verwendung von N-Methyl-O-pyrazol-

(4)yl-carbaminsäureester der Formel (I) zur Bekämpfung von Schädlingen.

7. Verfahren zur Herstellung von Schädlingsbekämpfungsmitteln, dadurch gekennzeichnet, dass man N-Methyl-O-pyrazol(4)yl-carbaminsäureester der Formel (I) mit Streckmitteln und/oder oberflächenaktiven Mitteln vermischt.

## Revendications

1. N-méthyl-carbamate de pyrazolyle(4) de formule (I):

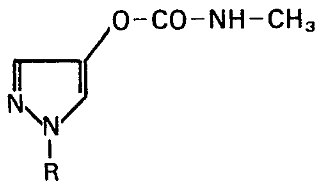

(I)

dans laquelle R est un radical alkyle, alcényle, alcynyle, alcoxyalkyle linéaire ou ramifié ayant à chaque fois jusqu'à 4 atomes de carbone dans le fragment alkyle ou un radical cycloalkyle ayant 3 à 6 atomes de carbone.

2. Composé selon la revendication 1, de formule

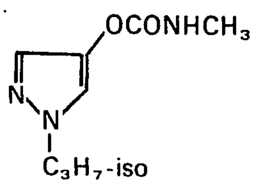

3. Composé selon la revendication 1, de formule

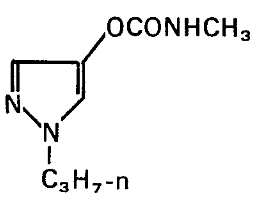

4. Procédé de production des esters d'acide carbamique de formule (I)

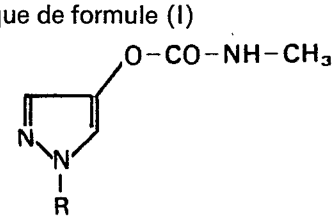

(I)

dans laquelle R est un radical alkyle, alcényle, alcynyle, alcoxyalkyle linéaire ou ramifié ayant à chaque fois jusqu'à 4 atomes de carbone dans le fragment alkyle ou un radical cycloalkyle ayant 3 à 6 atomes de carbone, caractérisé en ce que l'on introduit de façon usuelle le groupe N-méthylcarbamoyle dans des 4-hydroxypyrazoles de formule (II)

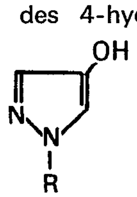

(II)

dans laquelle R a le sens précité.

5. Pesticide caractérisé en ce qu'il contient au moins un N-méthylcarbamate de pyrazolyle(4) de formule (I).

6. Application des N-méthylcarbamates de pyrazolyle(4) de formule (I) à la lutte contre les parasites et agents nuisibles.

7. Procédé de production de pesticides, caractérisé en ce qu'on mélange des N-méthylcarbamates de pyrazolyle(4) de formule (I) avec des agents d'allongement et éventuellement ou en variante avec des agents tensio-actifs.

## Patent Claims

1. N-Methyl-carbamic acid O-Pyrasol-4-yl esters of the formula (I)

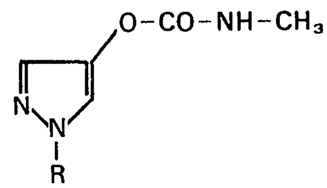

(I)

in which:
R represents straight-chain or branched alkyl, alkenyl, alkynyl or alkoxyalkyl with in each case up to 4 carbon atoms in the alkyl part, or cycloalkyl with 3-6 carbon atoms.

2. Compound according to claim 1 of the formula

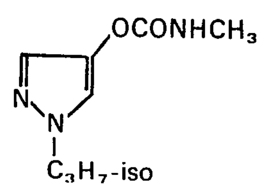

3. Compound according to claim 1 of the formula

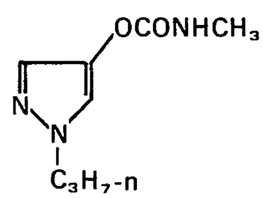

4. Process for the preparation of the carbamic acid esters of the formula (I)

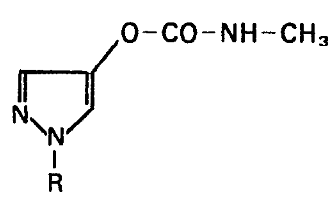

(I)

in which:
R represents straight-chain or branched alkyl, alkenyl, alkynyl, alkoxyalkyl with in each case up to 4 carbon atoms in the alkyl part, or cycloalkyl with 3-6 carbon atoms, characterised in that the N-methyl carbamoyl group is introduced in a conventional manner into 4-hydroxypyrazoles of the formula (II)

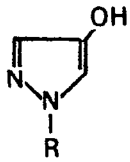

(II)

in which:
R has the meaning indicated above.

5. Agents for combating pests characterised in that they contain at least one N-methyl-carbamic acid O-pyrazol-4-yl ester of the formula (I).

6. Use of N-Methyl-carbamic acid O-pyrazol-4-yl esters of the formula (I) for combating pests.

7. Process for the preparation of agents for combating pests, characterised in that N-methyl-carbamic acid O-pyrazol-4-yl esters of the formula (I) are mixed with extenders and/or surface-active agents.